# EUROPEAN PATENT SPECIFICATION

(11) **EP 3 142 668 B1**
(45) Date of publication and mention of the grant of the patent: **03.06.2020**
(21) Application number: 15718626.3
(22) Date of filing: 31.03.2015
(51) Int. Cl.: A61K 31/519, A61K 36/41, A61P 15/00

(54) **A FORMULATION FOR AMELIORATING PREMATURE EJACULATION**
FORMULIERUNG ZUR LINDERUNG VON VORZEITIGER EJAKULATION
FORMULATION POUR AMÉLIORER L'ÉJACULATION PRÉCOCE

(30) Priority: 15.05.2014 IT RM20140243
(43) Date of publication of application: 22.03.2017
(73) Proprietor: IDI Integratori Dietetici Italiani S.r.l., 95020 Aci Bonaccorsi (CT) (IT)
(72) Inventor: BOTTINO, Pietro, I-95020 Aci Bonaccorsi (CT) (IT)
(74) Representative: Predazzi, Valentina
(86) International application number: PCT/IB2015/052361
(87) International publication number: WO 2015/173666

(56) References cited:
- DATABASE WPI Week 201311 Thomson Scientific, London, GB; AN 2013-A54353 XP002728336, & CN 102 697 035 A (GOU C) 3 October 2012 (2012-10-03)
- DATABASE WPI Week 201256 Thomson Scientific, London, GB; AN 2012-F18522 XP002728337, & CN 102 423 335 A (GUO K) 25 April 2012 (2012-04-25)
- DATABASE WPI Week 201169 Thomson Scientific, London, GB; AN 2011-M47275 XP002728338, & CN 102 172 384 A (TU C) 7 September 2011 (2011-09-07)
- DATABASE WPI Week 201045 Thomson Scientific, London, GB; AN 2010-H39067 XP002728339, & CN 101 721 615 A (ZHOU S) 9 June 2010 (2010-06-09)
- FARHATH KHANUM ET AL: "Rhodiola rosea: A Versatile Adaptogen", COMPREHENSIVE REVIEWS IN FOOD SCIENCE AND FOOD SAFETY, vol. 4, no. 3, 1 July 2005 (2005-07-01), pages 55-62, XP055133898, ISSN: 1541-4337, DOI: 10.1111/j.1541-4337.2005.tb00073.x

## Description

### TECHNICAL FIELD OF THE INVENTION

The present invention refers to a composition comprising as active ingredients *Rhodiola* and folic acid as claimed for use in the treatment of premature ejaculation. Moreover, the present invention also relates to said composition in the form of a pharmaceutical composition, nutraceutical composition, homeopathic composition, herbal composition.

### STATE OF THE PRIOR ART

Premature ejaculation (PE) is an ejaculation disorder characterized by excessive rapidity thereof. It is the most frequent male sexual pathology, with a >20% incidence in adult male population. Among sexual behavior dysfunctions the ejaculatory pathology certainly takes a relevant place because of its high incidence, controversial etiopathogenic hypotheses and because of the emotional impact it may cause. (Jannini EA, Lenzi A. 2006. Premature ejaculation: the impact on female and on the couple: the role of the endocrinologist. Porst H, Montorsi F. et al. The premature ejaculation prevalence and attitudes (PEPA) survey: prevalence, comorbidities and professional help-seeking. Eur Urol. 2007; 51:816-24).

Relevant epidemiological data on said pathology are reported by the PEPA survey (Premature Ejaculation Prevalence and Attitude; Porst H, Montorsi F. et al. The premature ejaculation prevalence and attitudes (PEPA) survey: prevalence, comorbidities and professional help-seeking. Eur Urol. 2007; 51:816-24) carried out on a sample of European (Germany and Italy) and U.S. males of age ranging from 18 to 70 years. The study demonstrates a main prevalence of PE equal to 22.7% which, moreover, proved similar in the various Countries considered (U.S.A. 24%; Germany 20.3%; Italy 20.0%).

PE definition can vary, but is typically characterized by a short Intravaginal Ejaculatory Latency (IELT) with concomitant sexual dissatisfaction and stress. An ejaculation occurring with minimal stimulation, during or soon after penetration, or before the subject desires it, is defined as Premature Ejaculation (PE) (DSM-IV-TR, 2000. Diagnostic and Statistical Manual of Psychiatric Disorders IV-Text Revision). Usually, premature ejaculation occurs nearly always within 30-60 s (80%) or between 1 and 2 min (20%). PE may be classified in primary (or lifelong, congenital) premature ejaculation, i.e. present right from the start of an individual's sexual history (i.e. from the first sexual experience), and secondary (or acquired) PE, i.e. manifesting itself from a time subsequent to the beginning of a sex life.

The most accredited theory at the basis of the onset of the PE disorder is represented by disorders of serotoninergic transmission at the central level. In particular, the neurotransmitter serotonin (5-Hydroxytryiptamine, 5-HT), during descending cerebral pathways, exerts an inhibitory-type modulation role on ejaculation, and a reduced concentration or failed functional effectiveness thereof would promote PE onset (Althof SE, Abdo CH et al. International Society for the Diagnosis and Treatment of Premature Ejaculation, J Sex Med. 2010;7:2947-69; Waldinger M, Berendsen HH et al. Premature ejaculation and serotoninergic antidepressants induced delayed ejaculation: The involvement of the serotoninergic system. Behav Brain Res. 1998; 92:111-8; Giuliano F. 5-Hydroxytryptamine in premature ejaculation: opportunities for therapeutic intervention. Trends Neurosci. 2007; 30:79-84).

Pharmacological treatment is the form of therapy most employed for premature ejaculation (Mohee A., Eardley I. Medical therapy for premature ejaculation. Ther Adv Urol. 2011. 3(5) 211-222). To date, dapoxetine is the sole drug approved for EP (Mondaini N, Fusco F et al. Dapoxetine treatment in patients with lifelong premature ejaculation: the reason of a "Waterloo". Urology. 2013; 82(3):620-4). Prior to dapoxetine approval, several were the pharmacological treatments used for the treatment of PE: topical treatments, creams or sprays causing penile desensitization, with a consequent ejaculation delay.

Moreover, traditional SSRIs (selective serotonin re-uptake inhibitors) were used off-label in the past for the treatment of PE following the observation that their use in the therapy of depression frequently caused, as a side effect, ejaculation delay (Waldinger MD. Premature ejaculation definition and drug treatment. Drugs 2007; 67(4):547-568). It should be pointed out that no conventional SSRI is approved for therapy of PE. Their use therefore remains still today off-label (Waldinger MD, Zwinderman AH et al. Relevance of methodological design for the interpretation of efficacy of drug treatment of premature ejaculation: A systematic review and meta-analysis. Int J Impot Res. 2004. 16:369-81).

However, treatment with the above-indicated drugs, i.e. SSRIs and dapoxetine, is associated with a set of side effects, among which fatigue, drowsiness, postural hypotension, nausea, dizziness, anxiety, vomiting, diarrhoea, dry mouth, excessive perspiration, effects which cause a high rate of patients drop-out (patients having poor compliance to the therapy). It follows that side effects and the high percentage of drop-out patients are the most important issues in the use of such drugs (Mondaini N, Fusco F et al. Dapoxetine treatment in patients with lifelong premature ejaculation: the reason of a "Waterloo". Urology. 2013; 82(3):620-4).

Various studies conducted on the species *Rhodiola Rosea* have demonstrated how it has the ability of aiding the organism to be more efficient against excessive stress and against some noxious substances and external agents (Ishaque S., Shamseer L. et al. Rhodiola Rosea for physical and mental fatigue: a systematic review. Complementary and Alternative Medicine. 2012. 12:70; Rhodiola Rosea: A Phytomedicinal Overview. Herbal Gram. 2002; 56:40-52). For this reason, *Rhodiola Rosea* was classified as "adaptogen". The adaptogenic properties of *Rhodiola* were mainly attributed to the ability of the same of influencing the levels and the activity of opioid monoamines and peptides such as beta-endorphins. It was observed that oral administration of *Rhodiola* extract to rats for 10 days modulates biogenic monoamines in the cerebral cortex, in the cerebral trunk and in the hypothalamus. In particular, in the cerebral cortex and in the cerebral trunk norepinephrine and dopamine levels drop, whereas serotonin levels increase substantially (Kelly GS. Rhodiola rosea: A Possible Plant adaptogen. Altern Med Rev. 2001. 6(3):293-302; Stancheva SL, Mosharrof A. Effect of the extract of Rodhiola Rosea L. on the content of the brain biogenic monoamines. Med Physiol. 1987. 40:85-87; Darbinyan V., Aslanyan G. et al. Clinical trial of Rhodiola rosea L. extract SHR-5 in the treatment of mild to moderate depression. Psychiatry. 2007. 61:343-348.

*Rhodiola Rosea* has been used for centuries in the traditional medicine to stimulate the Central Nervous System, to enhance physical and mental performance and to treat fatigue (Mannucci C1, Navarra M, Calzavara E, Caputi AP, Calapai G. Serotonin involvement in Rhodiola rosea attenuation of nicotine withdrawal signs in rats. Phytomedicine. 2012 Sep; 19(12): 1117-24). It is known that administration of *Rhodiola Rosea* extract elicits antidepressant activity (Chen QG, Zeng YS, et al. The effects of Rhodiola rosea extract on 5-HT level, cell proliferation and quantity of neurons at cerebral hippocampus of depressive rats. Phytomedicine. 2009;16(9):830-8). A clinical study on the treatment of depression with *Rhodiola Rosea* demonstrated an improvement of depression, insomnia, emotional instability and somatization of treated patients, without patients reporting side effects following administration (Darbinyan V., Aslanyan G. et al. Clinical trial of Rhodiola rosea L. extract SHR-5 in the treatment of mild to moderate depression. Psychiatry. 2007. 61:343-348). *Rhodiola* properties are ascribable to the presence of phenylpropanoid glycosides, in particular salidroside and rosavidine.

CN102697035 discloses a composition comprising *Rhodiola rosea* and yeast for strengthening sexual function.

CN102423335 discloses a composition comprising *Rhodiola rosea,* Hypericum and further components for the treatment of premature ejaculation.

CN102172384 discloses a composition comprising *Rhodiola rosea* and further components for the treatment of premature ejaculation.

CN101721615 discloses a composition comprising *Rhodiola rosea* and further components for the treatment of premature ejaculation.

Farath Khanum et al 2005, Comprehensive reviews in food science and food safety, vol 4 no.3 "Rhodiola rosea: a versatile adaptogen" discloses the use of *Rhodiola rosea* in the treatment of premature ejaculation.

Object of the present invention is to propose an effective alternative to the treatment of premature ejaculation, concomitantly allowing to overcome the drawbacks associated to the use of conventional therapies essentially based on administration of dapoxetine and on selective inhibitors of serotonin.

### SUMMARY OF THE INVENTION

The present invention refers to a composition comprising as active ingredients *Rhodiola* and folic acid for the treatment of premature ejaculation (PE) wherein the daily dosage of extract of *Rhodiola* is between 50 and 900 mg and the daily dosage of folic acid is between 50 and 400 µg.

The present invention stems from the observation that, as reported in the state of the art, premature ejaculation disorder finds in serotoninergic transmission disorders at the central level a possible etiology thereof.

As already indicated, to date the drugs used for the treatment of this pathology, i.e. dapoxetine and selective inhibitors of serotonin, though effective, entail a series of side effects which unavoidably limit their use.

The invention proposed herein aims at improving and controlling PE by the use of an alternative composition which has proved not only effective in the treatment of such a pathological condition, but characterized by less side effects compared to conventional therapeutic treatments. The innovation of the composition described herein lies in using as active ingredients (principles) two components of natural origin, i.e. *Rhodiola* and folic acid. In particular, the association of such components in the treatment of PE showed a series of advantages allowing a concrete use thereof in the medical field.
In particular, preliminary data demonstrated that the main advantages of the invention described herein are substantially represented by the fact that said composition:
- is a natural remedy for ameliorating and controlling premature ejaculation;
- interferes with Serotonin pathway, known to be one of the possible causes of PE;
- allows a treatment against PE which is effective, yet not invasive, not painful and without the side effects observed instead for pharmacological treatments such as those with dapoxetine and selective inhibitors of serotonin re-uptake (prescribed off-label);
- has a better patient compliance, with a reduction in the drop-out (scarce patient compliance to therapy) very frequently observed in patients subjected to conventional therapies.

Therefore, a first object of the present invention is a composition comprising *Rhodiola* and folic acid for use in the treatment of premature ejaculation.

In a second object of the invention, the composition comprises *Rhodiola* and folic acid, zinc and/or biotin.

In a third object of the invention, the composition comprises *Rhodiola* and folic acid, *Hypericum Perforatum* and/or 5-hydroxytryptophan.

In a fourth object of the invention, the composition comprises *Rhodiola* and folic acid, zinc, biotin, *Hypericum Perforatum* and/or 5-hydroxytryptophan.
A fifth object of the invention is a composition in accordance with the above-indicated objects, in the form of pharmaceutical composition, nutraceutical composition, homeopathic composition, herbal composition.

### GLOSSARY

- Ejaculation: emission, through the urethra, of seminal fluid (semen) caused by contractions of muscles at the basis of the penis and of epididymis, following the reaching of an orgasm.
- Premature Ejaculation (PE): ejaculation occurring with minimal stimulation, during or shortly after penetration, or sooner than desired by the subject.
- IELT: "Intravaginal Eiaculatorv Latency". intravaginal ejaculation latency time: the period of time intercurring between penetration start and ejaculatory phenomenon start.

### DETAILED DESCRIPTION OF THE INVENTION

The present invention refers to a composition comprising as active ingredients (principles) *Rhodiola* and folic acid, or consisting in said active ingredients as claimed.

*Rhodiola* is a genus of perennial plants belonging to the family of *Crassulaceae,* which appear as small shrubs having green leaves and inflorescences. In particular, for the purposes of the present invention, the *Rhodiola* species that can be used in the composition is *Rhodiola rosea.*

In one embodiment, *Rhodiola* is present in the composition in the form of extract.

By the term "extract", reference is made in this description to a set of substances of plant/natural origin obtained by extraction processes, from whole plants or parts thereof. Such processes are conventional and well-known to the average technician in the field, therefore a detailed description of processes for carrying out the invention described herein is unnecessary. Preferably, in case of *Rhodiola* the extract is a dry extract. Moreover, the extract is preferably obtained by water/ethanol extraction.

Merely by way of example *Rhodiola* extract may be prepared from the plant root.

In one embodiment of the invention, the *Rhodiola* extract is titrated at least at 0.5% of total rosavins. Preferably, the extract is titrated at 3% of total rosavins.

For the purposes of the treatment of premature ejaculation in accordance with the present invention, the amount of *Rhodiola* to be administered can range from about 50 to 900 mg per day. In that perspective, the composition can comprise a *Rhodiola* amount ranging from 50 to 900 mg, depending on administration posology. Hence, in other terms the composition can therefore comprise a *Rhodiola* amount of about 900 mg, of about 875 mg, of about 850 mg, of about 825 mg, of about 800 mg, of about 775 mg, of about 750 mg, of about 725 mg, of about 700 mg, of about 675 mg, of about 650 mg, of about 625 mg, of about 600 mg, 575 mg, of about 550 mg, of about 525 mg, of about 500 mg, 475 mg, of about 450 mg, of about 425 mg, of about 400 mg, of about 375 mg, of about 350 mg, of about 325 mg, of about 300 mg, of about 275 mg, of about 250 mg, of about 275 mg, of about 200 mg, of about 175 mg, of about 150 mg, of about 125 mg, of about 100 mg, of about 75 mg, of about 50 mg.

As indicated above, besides *Rhodiola,* folic acid is also present as active ingredient (principle) in the composition of the invention.

Folic acid is a B-group hydrosoluble vitamin and, since it is widely known and described in the state of the art, it needs no additional closer examination herein. Useful information on this vitamin can however be obtained from any biochemistry text, such as, e.g., "Lehninger Principles of Biochemistry" by David L. Nelson and Michael M. Cox.

Folic acid intervenes in the serotoninergic pathway; in fact, folate deficiency is associated with the reduction of Serotonin activity (5-HT) (Botez MI, Young SN et al. Folate deficiency and decreased brain 5-hydroxytryptamine synthesis in man and rat. Nature 1979; 278:182-3). In particular, folic acid provides the methyl group for methionine conversion to S-adenosylmethionine (SAM) and the latter influences 5-HT (serotonin) metabolism. Folic acid moreover has an important role in tetrahydrobiopterin synthesis, which is a limiting step in 5-HT synthesis. Without 5-MTHF (methyltetrahydrofolate) involvement in this process, SAM and neurotransmitter levels abate in cerebrospinal fluid. In addition, it was found that 5-MTHF distribution in the brain is similar to serotonin distribution (Korevaar WC, Geyer MA et al. Regional distribution of 5-methyltetrahydrofolic acid in brain. Nat New Biol. 1973; 245:244-5). Moreover, it was assessed that the neurogenic cause of PE may be both a hyposensitization of 5-HT2C receptors (receptors of serotonin subtype 2C) or a hypersensitization of 5-HT1A receptors (receptors of serotonin subtype 1A) (Waldinger MD. The neurobiological approach to premature ejaculation. J Urol 2002; 168:2359-67).

Therefore, it is known that folic acid intervenes in the Serotonin pathway, and that folic acid concentration in a group of patients with premature ejaculation was observed to be significantly lower than the control group (patients without PE (8.20 ± 3.76 ng/ml vs. 12.56 ± 4.35 ng/ml) (Yin TL, Yang J. et al. Folic acid supplementation as adjunctive treatment premature ejaculation. Medical Hypotheses. 2011. 76:414-416),

However, the Inventors of the present invention demonstrated that the use of said acid in association with *Rhodiola* is a valid, inexpensive, effective and acceptable alternative with respect to the use of serotonin re-uptake inhibitors (SSRIs). Moreover, advantageously folic acid contributes to improve the patient's psychological function, i.e. the patient's entire psychological situation such as, e.g., mood and anxiety improvement.

In accordance with the present invention any amount of folic acid, in the order of micrograms, milligrams or even grams, may be administered to a patient, as long as said dose is not toxic to the same patient. Preferably, the amount of folic acid to be administered is from 50 to 400 µg per day. In that perspective, the composition described herein can therefore comprise a folic acid amount of about 400 µg, of about 375 µg, of about 350 µg, of about 325 µg, of about 300 µg, of about 275, of about 250 µg, of about 275 µg, of about 200 µg, of about 175 µg, of about 150 µg, of about 125 µg, of about 100 µg, of about 75 µg, of about 50 µg, depending on the formulation type and the number of administrations envisaged per day.

In particular, a folic acid amount of about 200 µg per day is preferable, corresponding to 100% of NRVs (NRVs: daily nutrient reference values for vitamins and minerals (adults) pursuant to (EU) Regulation No. 1169/2011).

For the purposes of the present invention, any commercially available folic acid suitable for preparing the compositions described herein can be used.

In one embodiment of the invention, the *Rhodiola* extract : folic acid ratio is 100:1. In particular, the composition described can comprise 200 mg of *Rhodiola* extract and 200 µg of folic acid.

Moreover, the composition of the invention can further comprise as components also biotin and/or zinc. In other terms, the composition described herein can comprise *Rhodiola,* folic acid and biotin, or alternatively *Rhodiola,* folic acid and zinc, or even *Rhodiola,* folic acid, biotin and zinc.

Biotin is a B-group hydrosoluble vitamin which has the key role of cofactor for biologically crucial enzymes such as carboxylases, engaged in the synthesis of fatty acids and glucose through the gluconeogenesis process, and in the catabolism of some essential amino acids such as leucine, of cholesterol and of fatty acids.

The molecular activities of biotin macroscopically translate into a series of reactions useful for cell survival, and in particular the survival of those high-turnover tissues like the skin and related adnexa. The deficiency of this vitamin manifests itself with a series of symptoms, among which neurological symptoms like depression, lethargy and hallucinations. Biotin moreover fosters a normal functioning of the nervous system and a normal psychological function of the patient (Biotin. Alternative Medicine Review. 2007. Volume 12, Number 1). Biotin presence in the composition of the invention contributes to the maintaining of the patient's normal psychological function and limits any PE-related anxiety states of the same.

For the purposes of the present invention, any commercially available biotin suitable for preparing the compositions described herein can be used.

Any amount of biotin, in the order of micrograms, milligrams or even grams, may be administered to a patient, as long as said dose is not toxic to said patient. Preferably, the amount of biotin to be administered can range from 10 to 300 µg per day. In this perspective, the composition described herein can therefore comprise an amount of biotin of about 300 µg, of about 275, of about 250 µg, of about 275 µg, of about 200 µg, of about 175 µg, of about 150 µg, of about 125 µg, of about 100 µg, of about 75 µg, of about 50 µg, of about 25 µg, of about 20 µg, of about 10 µg, depending on the formulation type and the number of administrations envisaged per day.

In particular, a biotin amount of 50 µg per day is preferable, corresponding to 100% of NRVs (NRVs: daily nutrient reference values for vitamins and minerals (adults) pursuant to (EU) Regulation No. 1169/2011).

As already indicated above, the composition can also comprise zinc, both in association with the active principles (*Rhodiola* and folic acid) or optionally together with biotin.

For the purposes of the present invention, any commercially available zinc salt suitable for preparing the compositions described herein can be used. Zinc is preferably present in the composition in the form of zinc gluconate.

The amount of zinc to be administered in accordance to what described herein can range from 5 to 300 mg per day. In this perspective, the composition described herein can therefore comprise an amount of zinc of about 300 mg, of about 275 mg, of about 250 mg, of about 275 mg, of about 200 mg, of about 175 mg, of about 150 mg, of about 125 mg, of about 100 mg, of about 75 mg, of about 50 mg, of about 25 mg, of about 20 mg, of about 10 mg, of about 5 mg, depending on the formulation type and the number of administrations envisaged per day. In one embodiment of the invention, the composition comprises 10 mg of zinc gluconate.

In one embodiment of the invention, the composition comprises *Rhodiola* dry extract, zinc, folic acid and biotin. In particular, the composition may have, by way of example, the following composition:

| | | |
|---|---|---|
| - *Rhodiola* dry extract | 200 | mg (with a 6mg contribution of total rosavins); |
| - zinc gluconate | 10 | mg; |
| - folic acid | 200 | µg; |
| - biotin | 50 | µg. |

The composition of the invention, comprising, as already indicated above, *Rhodiola* and folic acid, and preferably biotin and/or zinc, can also comprise an extract of *Hypericum Perforatum.* Merely by way of example, the extract is a dry extract of *Hypericum Perforatum* that may be prepared from flowering tops, herb, flowers), i.e., *flos, herba c. floribus, summitas*) of the plant, and however from any portion/part thereof known to contain hypericin.

In one embodiment, *Hypericum Perforatum* extract is titrated at 0.3% hypericin.

Preferably, the amount of *Hypericum Perforatum* extract of the composition of the invention comprises no more than 0.7 mg of hypericin.

Moreover, the hyperforins/hypericin ratio preferably is not greater than 7. In said ratio, it should be considered that the hyperforins comprise both hyperforin and adiperforin of the extract.
As known, *Hypericum Perforatum* (Hypericum; St John's wort) is a plant having antidepressive properties. The active principles of Hypericum are hypericins, comprising: hypericin, pseudohypericin, cyclopseudohypericin, isohypericin and protohypericin. As reported in the literature, hypericum is capable of: inhibiting serotonin re-uptake at the post-synaptic level, inhibiting norepinefrine and dopamine uptake; inhibiting GABA re-uptake at the pre-synaptic level; increasing post-synaptic receptors for serotonin. On the basis of such data, it was hypothesized that hypericum could ameliorate premature ejaculation (Catherin A. Thomas, Shachi Tyagi et al. Effect of Hyperforin-Enriched Extract on Pro-Ejaculatory effect of 8-Hydroxy-2-(Di-N-Propylamino) Tetralin in Anesthetized Rats. Urology 70:813-816.2007

In particular, the amount of *Hypericum Perforatum* dry extract comprised in the composition of the invention can range from 20 mg to 700 mg. In particular, the amount of hypericum in the composition can be of about 700 mg, of about 675 mg, of about 650 mg, of about 625 mg, of about 600 mg, of about 575 mg, of about 550 mg, of about 525 mg, of about 500 mg, of about 475 mg, of about 450 mg, of about 425 mg, of about 400 mg, of about 375 mg, of about 350 mg, of about 325 mg, of about 300 mg, of about 275, of about 250 mg, of about 275 mg, of about 200 mg, of about 175 mg, of about 150 mg, of about 125 mg, of about 100 mg, of about 75 mg, of about 50 mg, of about 40 mg, of about 30 mg, of about 20 mg.

Preferably, hypericum dry extract further comprises a hypericin amount of ≤0.7 mg, i.e. of about 0.1 mg; 0.2 mg; 0.3 mg; 0.4 mg; 0.5 mg; 0.6 mg or 0.7 mg.

The composition described herein may further comprise also 5-hydroxytryptophan (5-HTP) amino acid. In particular, the presence of this amino acid in the composition allows to stimulate serotonin release by direct action on serotoninergic neurons, with entailed potentiation of the effectiveness due to the presence of *Rhodiola* and folic acid in the composition.

In particular, the amount of 5-hydroxytryptophan comprised in the composition of the invention can range from 50 mg to 500 mg. In particular, said amount can be of about 500 mg, of about 475 mg, of about 450 mg, of about 425 mg, of about 400 mg, of about 375 mg, of about 350 mg, of about 325 mg, of about 300 mg, of about 275, of about 250 mg, of about 275 mg, of about 200 mg, of about 175 mg, of about 150 mg, of about 125 mg, of about 100 mg, of about 75 mg, of about 50 mg.

Preferably, 5-HTP is provided in the composition described herein in the form of extracts of *Griffonia Simplicifolia.* In one embodiment of the invention, the *Griffonia Simplicifolia* extract comprised in the composition has a titration in 5-HT ranging from 20% to 99%.

The composition of the present invention may be formulated in any way deemed appropriate by the technician in the field for the purposes illustrated herein. In particular, the compositions can be formulated to foster an oral use thereof, and therefore may be in the form of capsule, tablet, beads, tablets, lozenge, chewing gum, granules, powder, syrup, elixir, hard gelatin, soft gelatin, suspension, emulsion, solution, etc.

In particular embodiments, the composition described herein may be made in the form of pharmaceutical composition, a nutraceutical composition, a homeopathic composition, a herbal composition.

In accordance with the present invention, the composition is homeopathic if, as also indicated in the Legislative Decree of April 24^{th}, 2006, N° 219, Art. 1, it is prepared according to a homeopathic production process described by the European Pharmacopoeia or, in the absence of such a description, from the Pharmacopoeias officially used in the States members of the European Community, providing the subjecting the substances of mineral or plant origin, or the synthetic substances, to dilution and dynamization (succussions). The composition of the invention is intended to be a nutraceutical composition when it is in the form, for instance, of food supplement or food for special medical purposes, or in general of any food in which to the nutritional components the active principles are associated, i.e. *Rhodiola* and folic acid and optionally the components biotin, zinc, hypericum, 5-HTP as previously described.

The nutraceutical composition may be made in solid, semi-solid, gelatinous or liquid form, like e.g. in the form of bars, candies, gelatins, beverages (suspensions or emulsions) or in the form of more complex foods, as long as their preparation does not entail a modification of the therapeutic effect at least of *Rhodiola* and folic acid present in the composition. An example of nutraceutical compositions is represented, e.g., by liquid beverages such as shakes, juices, or foods such as candies, biscuits, powders, granules, bars, etc.

The compositions according to the invention could therefore contain conventional excipients including, e.g., binding agents, like gum arabic, gelatine, sorbitol, gum tragacanth, and/or polyvinylpyrrolidone, cross-linked sodium carboxymethyl cellulose; fillers, like lactose, sugar, corn starch, rice starch, calcium phosphate, sorbitol and/or glycine; tableting lubricants, like magnesium stearate, talc, polyethylenglycol and/or silica; disintegrants, e.g. potato starch; wetting agents, like sodium laurylsulphate or granulated extracts of said plants; thickeners, like partially hydrogenated soybean oil, soybean oil; emulsifiers, like soy lecithin; strengthening agents like, e.g., glycerol; dyes; antiagglomerants like silicon bioxide, magnesium stearate; coloring agents like E171, etc. As mentioned above, all those excipients could be used in a pharmaceutically acceptable form. Evidently, the above-indicated excipients (categories and compounds) are by way of a non-limiting example within the realization of the present invention. The technician in the field could use other suitable excipients not punctually indicated herein with no addition of inventive activity. All those excipients commonly used by the technician in the field in the formulation of compositions of the above-indicated type are to be understood as encompassed within the realization and the of the invention. Moreover, the tablets, capsules, softgel capsules, gelatins, lozenges, etc., can be coated according to methods well known in the standard pharmaceutical practice. By way of example, the coating agents can be hydroxymethylcellulose, cellulose, stearic acid.

The composition could also be made in a liquid or semi-liquid form, as a suspension, emulsion, solution for oral administration, and could optionally contain natural aromatizing agents giving a palatable taste thereto.

The composition in the form of powder or granules could be pre-metered in suitable containers and ready for use, either by ingestion as such or to be resuspended in an appropriate liquid such as water, tea, etc. In this case as well, the composition could contain natural aromatizing agents giving a palatable taste thereto.

The composition according to any one of the embodiments described hereto could contain further active components, besides those present in the mixture of the invention, useful to assist PE treatment.

As previously indicated, the composition described herein proved effective in the treatment of premature ejaculation. In particular, by "treatment" it should be understood PE amelioration and control in patients affected by said pathology. The composition can be administered to patients in any one of its embodiments according to a posology that could be established by the doctor also on the basis of the patient's health conditions, weight and age. In a wholly exemplary way, the composition described herein may be administered for a treatment that may range from some days to some months. For instance, there may be envisaged a treatment of a patient with PE based on the administration of the composition in tablet formulation for a 90-day period (1 tablet per day).

It is understood, within the the present description, that in connection with the embodiments in which the components of the composition are indicated, the term "comprises/comprising" could also be replaced by the term "consists/consisting in".

Moreover, it is specified here that every time the present description provides a numerical range expressed in mg or µg, all integers and all decimal numbers up to two digits existing within this interval, extremes included, are understood to be punctually and explicitly described.

The present invention has hereto been described with reference to some embodiments thereof..

### CLINICAL STUDY

*"Tolerability and efficacy of a composition comprising Rhodiola rosea, folic acid, zinc and biotin in the management of patients with premature ejaculation: results from a phase I-II study".*

In particular, the composition subject of the study is:

| | |
|---|---|
| - *Rhodiola* dry extract | 200 mg; |
| - zinc | 10 mg; |
| - folic acid | 200 µg; |
| - biotin | 50 µg. |

### Study design:

Phase I-II - tolerability (main objective) and efficacy (secondary objective)

### Times:

Selection stage (1 month)
Enrolment (3 months)
Final analysis (1 month)

### Required sample size:

Single group: 86 patients (Reference: Johns Hopkins Bloomberg - School of Public Health).

### Main result measurement

Difference from baseline (PEDT questionnaire), patient-reported outcomes (PROs), after 3 months of therapy.

### Specific objectives

The main objective of the study is to:
- evaluate the tolerability to *Rhodiola rosea* extract in association with folic acid, biotin and zinc when administered to patients affected by lifelong (congenital) PE. The secondary objective is to:
   - evaluate the efficacy of *Rhodiola rosea* in association with folic acid, biotin and zinc when administered to patients affected by lifelong PE.

### Methodology

### Study design

In order to evaluate the tolerability and the efficacy of *Rhodiola* in association with folic acid, biotin and zinc in the management of patients with lifelong premature ejaculation, the following phase I-II study is conducted.

### Inclusion and exclusion criteria

All enrolled patients had the following characteristics:
- males >18 years and < 45 years
- affected by congenital PE, in accordance to what defined by the International Society for Sexual Medicine: premature ejaculation is a male sexual dysfunction characterized by ejaculation which always or nearly always occurs before or within about 1 minute of vaginal penetration; inability to delay ejaculation on all or nearly all vaginal penetrations; negative personal consequences, such as stress, apathy, frustration and/or avoidance of sexual intimacy (McMahon CG, Althof SE, Waldinger MD, et al. An evidence-based definition of lifelong premature ejaculation: report of the International Society for Sexual Medicine (ISSM) ad hoc committee for the definition of premature ejaculation. J Sex Med 2008;5:1590-606).
- absence of concomitant pharmacological therapy for ED= Erectile Dysfunction; LUTS= Lower Urinary Tract Symptoms
- absence of any suspect of secondary EP
- absence of major concomitant diseases
- absence of chronic therapy for depression (selective serotonin re-uptake inhibitors)

All enrolled patients were subjected to:
- collection of anamnestic data
- urological visit with DRE= Digital Rectal Examination
- IPSS, IIEF-15, PEDT, and SF-36. IPSS= International Prostate Symptom Score; IIEF-15= International Index of Erectile Function; PEDT= Premature Ejaculation Diagnostic Tool; SF-36= The 36-item Short Form Health Survey, and IELT= Intravaginal ejaculation latency time, which is particularly significant in the evaluation of any improvement in patients having premature ejaculation.
- patient-reported outcomes (PROs)
- urine examination with urine culture.

### Treatment mode

- the above composition was administered in the form of one tablet per day, in the morning, for 90 days. At time zero, information on demographic characteristics, medical history and the use of supplements/medicaments were collected

To all patients, one tablet/day of the aboveindicated composition was administered, in the morning, for 90 days. All patients were contacted by phone on day 30 of the therapy, in order to assess possible side effects and compliance with the treatment program. Two weeks after the end of the therapy, all patients were subjected to follow-up analysis by:
- urological visit
- IPSS, IIEF-15, PEDT, SF-36 and IELT
- patient-reported outcomes (PROs)
- urine examination with urine culture.

### Statistical analysis

Normal distribution of the variables was assessed using normal distribution of the variables using the Kolmogrov-Smirnov test, histograms, and p-p plots. If needed, the data were log (In) transformed to achieve a normal distribution. Data were analyzed based on the intention-to-treat approach. General characteristics of the study participants were expressed using descriptive statistics (means, SEM and ranges). For each independent value, changes from baseline were computed by subtracting the baseline value from the end-of trial value. Analysis of variance (ANOVA) was used for comparing means. Bonferroni adjustment test was used in a second stage of the analysis of variance. The sample size was calculated perspectively under the following conditions: difference between the groups =10% (reduction in PEDT questionnaire score), Alpha Error Level =0.05 two-sided, statistical power=80% and anticipated effect size (Cohen's d=0.5). The calculation yielded 72 individuals. Assuming a drop-out rate of nearly 20%, the patients enrolled in the study were 86.

### Obtained data

95 patients were enrolled (average age 32.3±5.6) and 91 of those were analyzed. Mena values in the various questionnaires were 1.,16±1.7, 26.10±2.9, 15.3±3.4, 97.1±0.9 for IPSS, IIEF-15, PEDT and SF-36, respectively. Mean IELT time before the study was of 73.67±46.9 sec. At the follow-up examination, 3 months after treatment, IPSS and IIEF had remained more or less constant - IPSS (1.45±1.5) and IIEF-15 (26.3±3.1) (p=0.19; p=0.64) whereas mean IELT time and quality of life measured with SF-36 questionnaire were significantly improved (p<0.001) with start- and end-of-study IELT values of 73.67±46.9 vs 102.36±60.0; p<0.001 and end-of-study SF-36 values of 98.2±0.5; p<0.001. Moreover, 55 out of 91 patients (60.4%) reported (PROs) an improvement in the control of the ejaculation.

To sum up, the study demonstrates that the composition of the invention is effective for the treatment of premature ejaculation.

## Claims

1. A composition comprising as active ingredients an extract of *Rhodiola* and folic acid for use in the treatment of premature ejaculation wherein the daily dosage of extract of *Rhodiola* is between 50 and 900 mg and the daily dosage of folic acid is between 50 and 400 µg.

2. The composition for use according to claim 1, wherein said *Rhodiola* is of the *Rhodiola rosea* species.

3. The composition for use according to claim 1 or 2, further comprising biotin and/or zinc.

4. The composition for use according to claim 3, wherein said zinc is zinc gluconate.

5. The composition according to any one of claims 1 to 4, , wherein the amount of *Rhodiola* extract is of 200 mg and the amount of folic acid is 200 µg.

6. The composition for use according to any one of claims 3 to 5, wherein the daily dosage of biotin is from 10 to 300 µg.

7. The composition for use according to any one of claims 3 to 6, wherein the daily dosage of zinc is from 5 to 300 mg.

8. The composition for use according to claim 7, wherein the amount of *Rhodiola* extract is 200 mg, the amount of folic acid is 200 µg, the amount of biotin is 50 µg and the amount of zinc is 10 mg

9. The composition for use according to any one of claims 1 to 8, further comprising 5-Hydroxytryptophan and/or an extract of *Hypericum Perforatum.*

10. The composition for use according to any one of claims 1 to 9, wherein said composition is a pharmaceutical composition, a nutraceutical composition, a homeopathic composition, a herbal composition.

11. The composition for use according to any one of claims 1 to 10, in the form of capsule, tablet, beads, tablets, lozenge, chewing gum, granules, powder, syrup, elixir, hard gelatin, soft gelatin, suspension, emulsion, solution.

12. The composition for use according to claim 10 or 11, wherein said nutraceutical composition is a food supplement or a food for special medical purposes.

## Patentansprüche

1. Zusammensetzung, die als aktive Wirkstoffe einen Extrakt aus Rhodiola und Folsäure zur Verwendung bei der Behandlung von vorzeitiger Ejakulation umfasst, wobei die Tagesdosis eines Extraktes aus Rhodiola zwischen 50 und 900 mg und die Tagesdosis von Folsäure zwischen 50 und 400 µg beträgt.

2. Zusammensetzung zur Verwendung gemäß Anspruch 1, wobei die Rhodiola aus Rhodiola rosea-Spezies besteht.

3. Zusammensetzung zur Verwendung gemäß Anspruch 1 oder 2, die weiterhin Biotin und/oder Zink umfasst.

4. Zusammensetzung zur Verwendung gemäß Anspruch 3, wobei das Zink in Form von Zinkglukonat vorliegt.

5. Zusammensetzung gemäß einem der Ansprüche 1 bis 4, wobei die Menge von Rhodiola-Extrakt 200 mg und die Menge von Folsäure 200 µg beträgt.

6. Zusammensetzung zur Verwendung gemäß einem der Ansprüche 3 bis 5, wobei die Tagesdosis von Biotin Werte von 10 bis 300 µg umfasst.

7. Zusammensetzung zur Verwendung gemäß einem der Ansprüche 3 bis 6, wobei die Tagesdosis von Zink Werte von 5 bis 300 µg umfasst.

8. Zusammensetzung zur Verwendung gemäß Anspruch 7, wobei die Menge von Rhodiola-Extrakt 200 mg, die Menge von Folsäure 200 µg, die Menge von Biotin 50 µg und die Menge von Zink 10 mg beträgt.

9. Zusammensetzung zur Verwendung gemäß einem der Ansprüche 1 bis 8, die weiterhin umfasst: 5-Hydroxytryptophan und/oder ein Extrakt aus Hypericum perforatum.

10. Zusammensetzung zur Verwendung gemäß einem der Ansprüche 1 bis 9, wobei die Zusammensetzung aus der folgenden Gruppe ausgewählt wird: eine pharmazeutische Zusammensetzung, eine nutrazeutische Zusammensetzung, eine homöopathische Zusammensetzung, eine pflanzliche Zusammensetzung.

11. Zusammensetzung zur Verwendung gemäß einem der Ansprüche 1 bis 10, deren Darreichungsform aus der folgenden Gruppe ausgewählt wird: Kapsel, Tablette, Kügelchen, Pillen, Dragee, Kaugummi, Granulat, Pulver, Sirup, Elixier, Hartgelatine, Weichgelatine, Suspension, Emulsion, Lösung.

12. Zusammensetzung zur Verwendung gemäß Anspruch 10 oder 11, wobei die nutrazeutische Zusammensetzung ein Nahrungsergänzungsmittel oder ein Lebensmittel für besondere medizinische Zwecke ist.

## Revendications

1. Composition comprenant, en tant que principes actifs, un extrait de *Rhodiola* et de l'acide folique, pour une utilisation dans le traitement de l'éjaculation précoce, dans laquelle la dose quotidienne d'extrait de *Rhodiola* est comprise entre 50 et 900 mg et la dose quotidienne d'acide folique est comprise entre 50 et 400 µg.

2. Composition pour une utilisation selon la revendication 1, dans laquelle ladite *Rhodiola* est de l'espèce *Rhodiola rosea.*

3. Composition pour une utilisation selon la revendication 1 ou 2, comprenant en outre de la biotine et/ou du zinc.

4. Composition pour une utilisation selon la revendication 3, dans laquelle ledit zinc est le gluconate de zinc.

5. Composition selon l'une quelconque des revendications 1 à 4, dans laquelle la quantité d'extrait de *Rhodiola* est de 200 mg et la quantité d'acide folique est de 200 µg.

6. Composition pour une utilisation selon l'une quelconque des revendications 3 à 5, dans laquelle la dose quotidienne de biotine est de 10 à 300 µg.

7. Composition pour une utilisation selon l'une quelconque des revendications 3 à 6, dans laquelle la dose quotidienne de zinc est de 5 à 300 mg.

8. Composition pour une utilisation selon la revendication 7, dans laquelle la quantité d'extrait de *Rhodiola* est de 200 mg, la quantité d'acide folique est de 200 µg, la quantité de biotine est de 50 µg et la quantité de zinc est de 10 mg.

9. Composition pour une utilisation selon l'une quelconque des revendications 1 à 8, comprenant en outre du 5-hydroxytryptophane et/ou un extrait de *Hypericum Perforatum.*

10. Composition pour une utilisation selon l'une quelconque des revendications 1 à 9, laquelle composition est une composition pharmaceutique, une composition nutraceutique, une composition homéopathique, une composition à base de plantes.

11. Composition pour une utilisation selon l'une quelconque des revendications 1 à 10, sous la forme d'une capsule, d'un comprimé, de perles, de comprimés, d'une pastille, d'une gomme à mâcher, de granules, d'une poudre, d'un sirop, d'un élixir, de gélatine dure, de gélatine molle, d'une suspension, d'une émulsion, d'une solution.

12. Composition pour une utilisation selon la revendication 10 ou 11, dans laquelle ladite composition nutraceutique est un complément alimentaire ou un aliment à des fins médicales particulières.
